# EUROPÄISCHE PATENTSCHRIFT

(11) **EP 1 632 223 B1**
(45) Veröffentlichungstag und Bekanntmachung des Hinweises auf die Patenterteilung: **09.10.2013**
(21) Anmeldenummer: 05090231.1
(22) Anmeldetag: 10.08.2005
(51) Int. Cl.: A61K 8/97, A61Q 19/00, A61Q 17/00, A61Q 1/00, A61Q 19/08, A61Q 17/04

(54) **Kosmetische Zusammensetzung zur Hautregenerierung**
Cosmetic composition for skin rejuvenation
Composition cosmétique de rajeunissement de la peau

(30) Priorität: 10.08.2004 DE 102004039459
(43) Veröffentlichungstag der Anmeldung: 08.03.2006
(73) Patentinhaber: Coty B.V., 2031 CC Haarlem (NL)
(72) Erfinder: Golz-Berner, Karin, 98000 Monaco (MC); Zastrow, Leonhard, 98000 Monaco (MC)
(74) Vertreter: Walter, Wolf-Jürgen

(56) Entgegenhaltungen:
- EP-A- 1 529 521
- FR-A- 2 779 058
- DATABASE WPI Section Ch, Week 200471 Derwent Publications Ltd., London, GB; Class A25, AN 2004-726625 XP002355256 & RU 2 236 842 C1 (SVOBODA COSMETICS ASSOC STOCK CO) 27. September 2004 (2004-09-27) & RU 2 236 842 C1 27. September 2004 (2004-09-27)
- DATABASE WPI Section Ch, Week 199913 Derwent Publications Ltd., London, GB; Class B04, AN 1999-148433 XP002355728 & JP 11 012122 A (POLA CHEM IND INC) 19. Januar 1999 (1999-01-19)
- DATABASE WPI Section Ch, Week 200413 Derwent Publications Ltd., London, GB; Class B04, AN 2004-126217 XP002355257 & JP 2003 277226 A (NOEVIR KK) 2. Oktober 2003 (2003-10-02)
- PATENT ABSTRACTS OF JAPAN Bd. 1999, Nr. 03, 31. März 1999 (1999-03-31) & JP 10 316529 A (SHINKAI TERUO), 2. Dezember 1998 (1998-12-02)

## Beschreibung

Die Erfindung betrifft eine kosmetische Zusammensetzung, enthaltend einen Wirkkomplex aus Pflanzenextrakten zur Regenerierung der Haut.

Aus verschiedenen Veröffentlichungen ist der Einsatz von Sojaextrakten bekannt, z.B. zur Verhinderung von UV-Schäden (US 6,455,032, zusammen mit Isoflavonen), zur Aknebehandlung (US 2003/0224075 A1) und als Zusatz in Form von Proteinkonzentrat zu wasserfreien kosmetischen Produkten wie Lippenstiften, Nagellacken etc. (EP0948309 zusammen mit hydrolysierter Stärke). Aus FR 2779058 A1 ist die Verwendung von Saponin/Sapogenin aus Soja oder Luzerne zur Kollagen-IV-Bildung bekannt. JP 11-012122 A offenbart ein Hautpflegemittel aus Ursolsäure oder einem oder mehreren Pflanzenextrakten, darunter Kornblumenextrakt, und einer entzündungswidrigen Substanz aus weiteren Pflanzenextrakten.

Der Erfindung liegt die Aufgabe zugrunde, eine Zusammensetzung mit einem Wirkkomplex auf pflanzlicher Basis bereitzustellen, der eine besonders langanhaltende Hautschutz- und Hautregenerierungswirkung aufweist.

Eine weitere Aufgabe besteht in der Bereitstellung einer Zusammensetzung mit einem Wirkkomplex, der die Hautelastizität verbessert, zu einer deutlichen Verringerung der Faltentiefe führt, entzündungshemmend wirkt und den Sonnenschutzfaktor SPF erhöht.

Erfindungsgemäß bereitgestellt wird eine kosmetische Zusammensetzung, enthaltend einen Wirkkomplex zur Hautregenerierung, der aus 5 - 20 Gew-% eines Extraktes von Centaurea cyanus und 80 - 95 Gew-% eines Extraktes von reifen Sojabohnen besteht, bezogen auf das Gesamtgewicht des Komplexes, wobei das der Komplex zusammen mit einem Trägerstoff, Hilfsstoffen oder Gemischen davon vorliegt. Der Trägerstoff kann Wasser sein. Ein bevorzugter Anteil des Komplexes in der kosmetischen Zusammensetzung liegt im Bereich von 0,1 - 20 Gew-%, bezogen auf das Gesamtgewicht der Zusammensetzung.

Besonders bevorzugt liegt der Wirkkomplex mit einem Anteil von 0,1 - 7 Gew-% vor, bezogen auf das Gesamtgewicht der kosmetischen Zusammensetzung.

Es wurde gefunden, dass der erfindungsgemäße Wirkkomplex bei in vitro-Tests eine deutlich niedrigere Verringerung des ATP-Spiegels in der Zelle zulässt, wenn eine 0,1 %ige Lösung vor einer UV-Bestrahlung auf die Haut aufgetragen wird. Selbst bei der genannten geringen Konzentration des Wirkkomplexes von 0,1 % liegt der ATP-Spiegel ca. 13 % über dem einer Kontrollprobe. Die DNA-Reparaturrate liegt ebenfalls etwa 10-15 % über der natürlichen Reparaturrate bei in vitro-Tests mit unterschiedlich starker UV-Strahlung.

Weiterhin wurde bei in vivo-Tests gefunden, dass die Hautelastizität um 15-25 % verbessert wird nach 3-6-wöchiger Behandlung mit einer Formulierung, die 2 Gew-% des Wirkkomplexes enthält. Über den gleichen Zeitraum mit einer Formulierung, die 5 Gew-% des Wirkkomplexes enthält, wird die Faltentiefe bei sog. Krähenfüßen am Auge in vivo gegenüber Placebos um 100 - 400 % verringert.

Das Verhältnis von Centaurea-Extrakt : Soja-Extrakt liegt vorzugsweise im Bereich 2 - 10 : 90 - 98.

Darüber hinaus hat der erfindungsgemäße Wirkkomplex eine unerwartet starke anti-inflammatorische Wirksamkeit, die bereits bei Konzentrationen unter 1 Gew-% signifikante Verbesserungen von bis zu 30 % gegenüber einer Kontrollprobe erkennen lässt.

Es wurde gefunden, dass eine Hemmung der Synthese von Prostaglandin E2 (PGE₂)auftritt. Diese Hemmung wird ausschließlich durch die Kombination von Kornblumenextrakt mit Sojaextrakt getragen, während der Sojaextrakt allein keine signifikante Hemmwirkung zeigt (Kontrolle:100; 0,1 % Sojaextrakt 99; 0,5 % Sojaextrakt 99; 0,1 % Sojaextrakt/Kornblumenextrakt (90:10) 88; 0,5 % Sojaextrakt/Kornblumenextrakt (90:10) 69).

Obwohl Wirkungen von Soja bzw. Sojaextrakten in Bezug auf die Haut als Erweichungsmittel oder auch zur Sebumbeeinflussung bekannt sind, war es doch überraschend, dass eine Kombination allein mit Kornblumenextrakten einen deutlichen Schutz gegenüber UV-Strahlung zeigt und dieser Schutz für einen längeren Zeitraum anhält. Unter "längerem Zeitraum" wird eine Nachwirkung für 12-14 Tage über einen Behandlungszeitraum von 6 Wochen hinaus verstanden, ohne dass eine weitere Hautbehandlung stattfindet und bei einem Wirkungsverlust von nicht mehr als 20 - 25 %.

"Schutz gegenüber UV-Strahlung" bedeutet eine Erhöhung des Sonnenschutzfaktors SPF auf Basis bekannter UV-Filter von z.B. SPF 4 auf SPF 7 infolge der Anwesenheit einer 2,5 %igen Lösung des Wirkkomplexes innerhalb einer Sonnenschutzzubereitung (SPF = Sun Protection Factor).

Der erfindungsgemäße Wirkkomplex ist kein Komplex im rein chemischen Sinne, sondern ein Gemisch verschiedener Bestandteile, deren Zusammenwirken eine bisher nicht bekannte und nicht vorhersehbare Wirkung oder einen solchen Aspekt einer Wirkung zeigt. Der Komplex geht damit über die Summe der Einzelwirkungen der Bestandteile hinaus.

Extraktgrundlage für Centaurea sind die oberirdischen Teile der Pflanze, vorzugsweise die Blüten. Die Extraktion erfolgt auf wässriger Basis ohne organische Lösungsmittel und wird bei Umgebungstemperatur für 4 - 36 h durchgeführt, so dass die hydrophilen Bestandteile der Pflanze erhalten bleiben.

Die frisch geernteten und gegebenenfalls gelagerten Sojabohnen werden ohne weitere Vorbehandlung, wie Trocknung oder hydrolytische Spaltung, auf wässriger Basis ohne organische Lösungsmittel kalt (Umgebungstemperatur) über einen Zeitraum von 12-48 Stunden extrahiert zwecks Erhalt der hydrophilen Bestandteile der Pflanze.

Der erfindungsgemäße Wirkkomplex kann in bestimmten kosmetischen Anwendungsformen von Zusammensetzungen eingesetzt werden. Derartige Anwendungsformen sind z.B. Lotion, Gel, Tagescreme, Nachtcreme, Sonnencreme, Sonnenmilch, After-sun Produkt, Spray, Balm, Maske, Makeup, Handcreme, Körperpflegeprodukt, Haarmaske.

Die Konzentration des Wirkkomplexes in der entsprechenden Anwendungsform liegt bei 0,1 bis 7 Gew-%, vorzugsweise 0,1 bis 5 Gew-%, besonders bevorzugt 0,5 bis 4 Gew-%.

Die Zusammensetzung enthält neben dem Wirkkomplex kosmetische Hilfs- und Trägerstoffe, wie sie üblicherweise in solchen Zubereitungen verwendet werden, z.B. Wasser, Konservierungsmittel, Farbstoffe, Pigmente mit färbender Wirkung, Verdickungsmittel, Duftstoffe, Alkohole, Polyole, Ester, Elektrolyte, Gelbildner, polare und unpolare Öle, Polymere, Copolymere, Emulgatoren, Wachse, Stabilisatoren.

Es können auch zusätzliche Wirkstoffe neben dem Wirkkomplex eingesetzt werden. Zu diesen kosmetischen Wirkstoffen gehören z.B. anorganische und organische Lichtschutzmittel, Radikalfänger, Feuchthaltemittel, Vitamine, Enzyme, weitere pflanzliche Wirkstoffe, Polymere, Antioxidationsmittel, weitere entzündungswidrige natürliche Wirkstoffe, mit Sauerstoff beladene asymmetrische lamellare Aggregate gemäß WO 94/00109, Aufschlußprodukte von Hefen oder pflanzlichen Stoffen, hergestellt durch ein schonendes Ultraschall-Aufschlußverfahren gemäß WO 94/13783, Kaolin sowie mit SiO₂ modifiziertes Kaolin gemäß WO94/17588.

Zu Antioxidationsmitteln gehören Vitamine wie Vitamin C und Derivate davon, beispielsweise Ascorbylacetate, -phosphate und - palmitate; Vitamin A und Derivate davon; Folsäure und deren Derivate, Vitamin E und deren Derivate, wie Tocopherylacetat; Superoxiddismutase; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure.

Es ist weiterhin vorteilhaft, den kosmetischen Zusammensetzungen mit dem erfindungsgemäßen Wirkkomplex entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxy-benzophenon; 3-Benzylidencampher-Derivate wie 3-Benzylidencampher.

Wasserlösliche UVB-Filter sind z.B. Sulfonsäurederivate von Benzophenon oder von 3-Benzylidencampher oder Salze wie das Na-oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bos-Benzotriazolyl Tetramethylbutylphenol, Disodium Phenyl Dibenzimidazole Tetrasulfonate, Bis-Ethylhexyloxyphenol Methoxyphenyl Triazine.

Weiterhin einsetzbar als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch verwendet werden können.

Besonders bevorzugt als anorganische Pigmente sind agglomerierte Substrate von TiO₂ und/oder ZnO, die einen Gehalt an sphärischen und porösen SiO₂-Teilchen aufweisen, wobei die SiO₂-Teilchen eine Teilchengröße im Bereich von 0,05 µm bis 1,5 µm haben, und neben den SiO₂-Teilchen andere anorganische teilchenförmige Stoffe mit sphärischer Struktur vorliegen, wobei die sphärischen SiO₂-Teilchen mit den anderen anorganischen Stoffen definierte Agglomerate mit einer Teilchengröße im Bereich von 0,06 µm bis 5 µm bilden (gemäß WO99/06012).

Ein weiterer bevorzugter Wirkstoffzusatz für das erfindungsgemäße Kosmetikum ist eine Zubereitung mit hohem Radikalschutzfaktor (siehe WO99/66881) mit einem Gehalt an einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser. Diese Zubereitung kann gegebenenfalls noch ergänzt sein durch ein Superoxiddismutase-haltiges Hefeaufschluss-produkt und/oder Cyclodextrine (WO 94/13783).

Die für die Erfindung eingesetzten Öle können übliche kosmetische Öle sein, wie ein Mineralöl; hydriertes Polyisobuten , synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Besonders geeignete Öle sind beispielsweise Siliconöle, Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether sowie pflanzliche Öle, wie Calendulaöl, Jojobaöl, Avocadoöl, Macadamianußöl, Rizinusöl, Kakaoobutter, Kokosnußoil, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinußöl, Distelöl und Gemische davon.

Je nachdem welche Öle ausgewählt werden, werden die kosmetischen Eigenschaften der festen Zusammensetzung beeinflußt, wie Transparenzgrad, Weichheit, Härte, Spreitungswirkung.

Als Ester oder Ether sind zum Beispiel geeignet Dipentaerythrityl hexacaprilate/hexacaprate/tridecyl trimellitate/tridecyl stearate/neopentyl glycol dicaprylate dicaprate, Propylene glycol dioctanoate, Propylene glycol dicaprylate 2,30 dicaprate, Tridecyl stearate/neopentyl glycol dicaprylate dicaprate/tridecyl trimellitate, Neopentyl glycol dioctanoate, Isopropyl myristate, Diisopropyl dimer dilinoleate, Trimethylpropane triisostearate, Myristyl ether, Stearyl ether, Cetearyl octanoate, Butyl ether, Dicaprylyl ether, PPG1-PEG9 Lauroyl glycol ether, PPG15 Stearyl ether, PPG14 Butyl ether, Fomblin HC25.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure etc.

Als Feuchthaltemittel sind bevorzugt Glycerin, Butylenglycol, Propylenglycol und Gemische davon.

Für die Emulsionsbildung können als oberflächenaktive Mittel anionische, amphotere, nichtionische oder kationische oberflächenaktive Mittel oder Gemische davon eingesetzt werden.

Die Erfindung soll nachstehend durch Beispiele näher erläutert werden. Alle Angaben erfolgen in Gewichtsprozent, sofern nichts anderes angegeben ist.

In der dazugehörigen Zeichnung zeigen
Fig. 1: Säulendiagramm Schutz gegen ATP-Verringerung nach UV-Bestrahlung (control = Kontrollprobe; 0,1 % Phytoperfect = 0,1 %iger Wirkkomplex Kornblume : Soja = 1 : 9 in Wasser)
Fig. 2: Säulendiagramm UV-induzierte Erhöhung der Elastase-Aktivität (untreated control = unbehandelte Kontrollprobe; L-Phytoperfect = 5 %iger Wirkkomplex; Ordinate 100 % = Elastase-Aktivität der unbehandelten und unbestrahlten Flächen).

### Beispiel 1 After Sun Creme I

| **Phase A** | |
|---|---|
| Wasser | q.s. ad 100 |
| Glycerin | 4,0 |

| **Phase B** | |
|---|---|
| Dimethicone/Crosspolymer Dimethicone PEG 10 Dimethicone Crosspolymer PEG 15 Dimethicone | 3,5 |
| Crosspolymer | 6,2 |
| Silicone | 4,5 |

| **Phase C** | |
|---|---|
| Ethanol | 5,0 |
| RPF-Komplex¹ | 0,5 |
| Kamillenextrakt | 2,0 |
| Extraktgemisch Kornblume:Soja 10:90 | 5,0 |
| Konservierungsmittel | 1,0 |
| Parfümöl | 0,2 |

| | |
|---|---|
| ¹ gemäß WO99/66881 Beispiel 1 | |

Phase B und A werden auf 75°C erwärmt und unter Rühren zusammengeführt. Das Gemisch wird wenigstens 20 min homogenisiert und auf 35°C abgekühlt. Anschließend erfolgt unter Rühren der Zusatz von Phase C.

### Beispiel 2 After Sun Creme II

Es wird wie in Beispiel 1 gearbeitet, wobei der RPF-Komplex nicht enthalten war.

### Beispiel 3 Makeup I

| **Phase A** | |
|---|---|
| Wasser | q.s. ad 100 |
| Glycerin | 4,0 |
| Ammonium Acryloyl-dimethyltaurate VP/Copolymer | 2,0 |

| **Phase B** | |
|---|---|
| PEG 20 Methylglucose Sesquistearate-Tocopherol | 3,0 |
| Petrolatum | 1,5 |
| Squalane | 1,0 |
| Silicone | 5,0 |

| **Phase C** | |
|---|---|
| Ethanol | 2,0 |
| Pigmente | 3,5 |
| Kaolin modifiziert nach ² | 1,5 |
| RPF-Komplex¹ | 0,5 |
| Konservierungsmittel | 0,8 |
| Parfüm | 0,5 |
| Extraktgemisch Kornblume:Soja 10:90 | 2,0 |

| | |
|---|---|
| ² WO96/17588 Beispiel 1 | |

Es wurde wie im wie Beispiel 1 gearbeitet.

### Beispiel 4 Makeup II

Es wurde die Zusammensetzung gemäß Beispiel 3 hergestellt ohne den RPF-Komplex.

### Beispiel 5 Körpergel

| **Phase A** | |
|---|---|
| Wasser | q.s. ad 100 |
| Carbomer | 2,0 |
| Ethanol | 1,5 |
| Glycerin | 2,5 |

| **Phase B** | |
|---|---|
| Triethanolamin | 1,5 |
| Silicon | 5,0 |
| RPF-Komplex¹ | 0,5 |
| Extraktgemisch Kornblume:Soja 18:82 | 0,5 |
| Konservierungsmittel | 0,5 |
| Parfüm | 0,5 |

Bei Raumtemperatur wurden beide Phasen miteinander vermischt und anschließend homogenisiert.

### Vergleichsbeispiel 1 Schutz gegen ATP-Verringerung nach UV-Bestrahlung

Es wurde der ATP-Spiegel nach UV-Bestrahlung von menschlichen Keratinocyten beurteilt. Das Zellwachstum erfolgte in Gegenwart von 5% FCS. Die Messung erfolgte bei 12 Probanden mit einer Basisformulierung ohne Zusatz von Lichtschutzfiltern entsprechend Beispiel 3. Die Kontrollprobe enthielt kein Extraktgemisch; die Wirkstoffprobe enthielt 0,1 Gew-% Extraktgemisch mit einem Verhältnis Centaurea cyanus : Sojabohne 18 : 82.

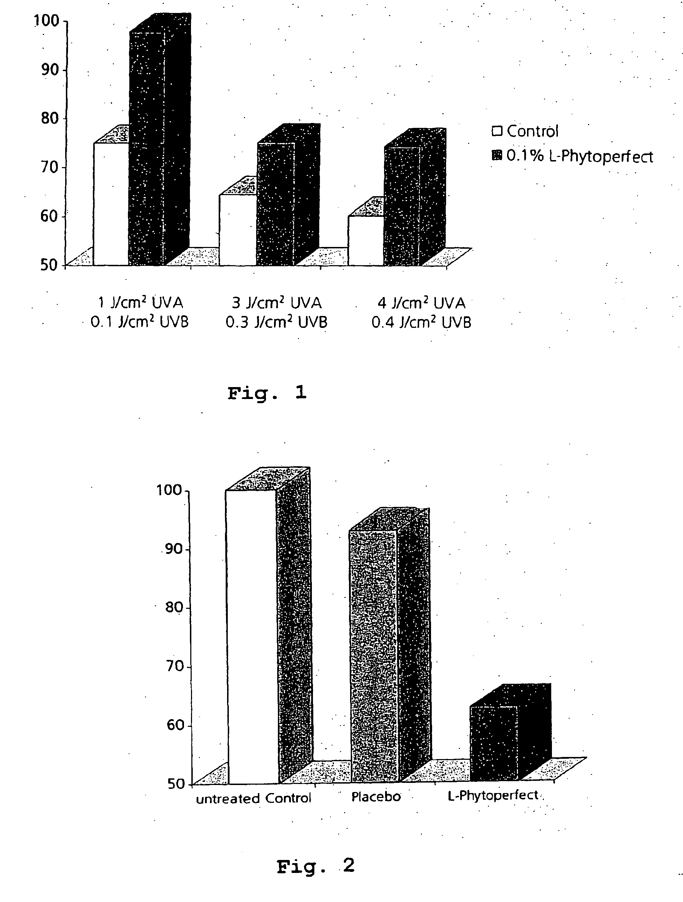

Bestrahlt wurde mit drei unterschiedlichen Dosen UVA/UVB-Strahlung, wie in Fig. 1 angegeben.
Der Schutz gegen ATP-Verringerung bei einer Bestrahlung UVA/UVB 1 J/cm²
UVA/UVB 0,1 J/cm² lag um ca. 31 % höher als bei der Kontrollprobe;
UVA/UVB 3 J/cm²
UVA/UVB 0,3 J/cm² lag um ca. 13 % höher als bei der Kontrollprobe;
UVA/UVB 4 J/cm²
UVA/UVB 0,4 J/cm² lag um ca. 20 % höher als bei der Kontrollprobe.

### Vergleichsbeispiel 2 Elastizitätsaktivität

Es wurde der Einfluß auf die UV-induzierte Erhöhung der Elastase-Aktivität untersucht. Eine Formulierung gemäß Beispiel 1 mit 5 Gew-% des Komplexes (Verhältnis Centaurea cyanus : Sojabohne = 1 : 9) wurde von 5 Probanden zweimal täglich auf ein Hauareal aufgetragen. Daneben wurde ein Placebo mit der Basisformulierung von Beispiel 1 (ohne den Wirkkomplex) aufgetragen. Nach einer Woche wurde die behandelte und unbehandelte Haut UV-bestrahlt und die Elastase-Aktivität bestimmt.

Es ergaben sich folgende Werte, die in Fig. 2 dargestellt sind:

| | |
|---|---|
| Unbehandelte Haut | 100 % |
| Behandelte Haut mit Placebo | 92 % |
| Behandelte Haut mit Formulierung von Bsp. 1 | 64 %. |

Eine spätere Versuchsreihe mit 2 Gew-% des Komplexes ergab einen Wert von 78 %, der sich damit gut in eine abfallende Kurve einordnet und der die Langzeitwirkung deutlich macht.

## Patentansprüche

1. Kosmetische Zusammensetzung zur Hautregenerierung, **dadurch gekennzeichnet, dass** sie umfasst: einen Wirkkomplex aus 5 - 20 Gew-% eines Extraktes von Centaurea cyanus und 80 - 95 Gew-% eines Extraktes der Samen der Sojabohne, bezogen auf das Gesamtgewicht des Wirkkomplexes, und wobei der Wirkkomplex zusammen mit Trägerstoffen, Hilfsstoffen oder Gemischen davon in der Zusammensetzung vorliegt.

2. Zusammensetzung nach Anspruch 1, **dadurch gekennzeichnet, dass** der Wirkkomplex in einer Konzentration von 0,1 - 20 Gew-% in der Zusammensetzung vorliegt, bezogen auf das Gesamtgewicht der Zusammensetzung.

3. Zusammensetzung nach Anspruch 2, **dadurch gekennzeichnet, dass** der Wirkkomplex in der Zusammensetzung mit einem Anteil von 0,1 - 7 Gew-% vorliegt.

4. Zusammensetzung nach Anspruch 3, **dadurch gekennzeichnet, dass** der Wirkkomplex in der Zusammensetzung mit einem Anteil von 0,5-5 Gew-% vorliegt.

5. Zusammensetzung nach Anspruch 1, **dadurch gekennzeichnet, dass** sie aus der Gruppe ausgewählt ist bestehend aus Lotion, Creme, Spray, Balm, Gel, Sonnenmilch, After-sun-Produkt.

6. Zusammensetzung nach Anspruch 1, **dadurch gekennzeichnet, dass** sie einen weiteren Wirkstoff enthält, ausgewählt aus der Gruppe, bestehend aus Antioxidationsmitteln, Vitaminen, Kreatin, UV-Filtern, Superoxiddimutase, Kaolin, mit Sauerstoff beladenen asymmetrischen lamellaren Aggregaten und Gemischen davon.

7. Zusammensetzung nach Anspruch 1, **dadurch gekennzeichnet, dass** das Verhältnis von Centaurea-Extrakt zu Sojaextrakt im Bereich von 2 bis 10 : 90 bis 98 liegt, bezogen auf das Trockengewicht.

8. Zusammensetzung nach Anspruch 1 zur Anwendung in einem Verfahren zur Hautregenerierung.

9. Zusammensetzung nach Anspruch 1 zur Anwendung in dem Verfahren nach Anspruch 8 zur Vorbereitung der Haut bei nachfolgender UV-Bestrahlung.

10. Verwendung der kosmetischen Zusammensetzung nach Anspruch 1 zur Erhöhung des Sonnenschutzfaktors SPF.

## Claims

1. Cosmetic composition for skin regeneration, which comprises an effective complex from 5 to 20 % by weight of an extract of Centaurea cyanus and 80 to 95 % by weigth of an extract from the seed of soy bean, related to the total weight of the effective complex, and wherein the effective complex is present in the composition with carriers, excipients or mixtures thereof.

2. Composition according to claim 1, wherein the effective complex is present in the composition in a concentration of 0.1 to 20 % by weight related to the total weight of the composition.

3. Composition according to claim 2, wherein the effective complex is present in the composition in a concentration of 0.1 to 7 % by weight.

4. Composition according to claim 3, wherein the effective complex is present in the composition in a concentration of 0.5 to 5 % by weight.

5. Composition according to claim 1, wherein the composition is selected from the group consisting of a lotion, cream, spray, balm, gel, sun milk, after-sun product.

6. Composition according to claim 1, wherein the composition contains a further active substance selected from the group consisting of antioxidants, vitamins, creatine, UV filters, superoxide dismutase, kaolin, asymmetric lamellar aggregates loaded with oxygen and mixtures thereof.

7. Composition according to claim 1, wherein the ratio of Centaurea extract to soy extract is in the range of 2 to 10 : 90 to 98 relative to the dry weight.

8. Composition according to claim 1 for use in a skin regeneration process.

9. Composition according to claim 1 for use in a process according to claim 8 for preparing the skin for subsequent UV irradiation.

10. Use of the cosmetic composition according to claim 1 for increasing the sun protective factor (SPF).

## Revendications

1. Composition cosmétique de rajeunissement de la peau, **caractérisée en ce qu'**elle comprend : un complexe actif de 5 à 20 % en poids d'un extrait de *Centaurea cyanus* et de 80 à 95 % en poids d'un extrait de graines de soja par rapport au poids total du complexe actif, et dans laquelle le complexe actif se présente dans la composition, associé à des véhicules, des adjuvants ou des mélanges de ceux-ci.

2. Composition selon la revendication 1, **caractérisée en ce que** le complexe actif se présente en une concentration de 0,1 à 20 % en poids dans la composition, par rapport au poids total de la composition.

3. Composition selon la revendication 2, **caractérisée en ce que** le complexe actif se présente dans la composition en une proportion de 0,1 à 7 % en poids.

4. Composition selon la revendication 3, **caractérisée en ce que** le complexe actif se présente dans la composition en une proportion de 0,5 à 5 % en poids.

5. Composition selon la revendication 1, **caractérisée en ce qu'**elle est choisie dans le groupe constitué des lotions, des crèmes, des sprays, des baumes, des gels, du lait solaire, des produits après-soleil.

6. Composition selon la revendication 1, **caractérisée en ce qu'**elle contient une autre substance active choisie dans le groupe constitué des antioxydants, des vitamines, de la créatine, des filtres UV, du superoxyde dismutase, du kaolin, des agrégats lamellaires asymétriques chargés d'oxygène et de leurs mélanges.

7. Composition selon la revendication 1, **caractérisée en ce que** le rapport de l'extrait de centaurée à l'extrait de soja est de l'ordre de 2 à 10 : 90 à 98 par rapport au poids sec.

8. Composition selon la revendication 1, pour son utilisation dans un procédé de rajeunissement de la peau.

9. Composition selon la revendication 1, pour son utilisation dans le procédé selon la revendication 8, pour la préparation de la peau à une irradiation UV consécutive.

10. Utilisation de la composition cosmétique selon la revendication 1, pour augmenter le facteur de protection solaire SPF.
